# EUROPEAN PATENT SPECIFICATION

(11) **EP 2 007 287 B1**
(45) Date of publication and mention of the grant of the patent: **29.06.2016**
(21) Application number: 07722546.4
(22) Date of filing: 30.03.2007
(51) Int. Cl.: A61B 10/02

(54) **TISSUE SAMPLE COLLECTION SYSTEM WITH VISUAL SAMPLE INSPECTION**
SYSTEM ZUR SAMMLUNG VON GEWEBEPROBEN MIT VISUELLER PROBENINSPEKTION
SYSTÈME DE PRÉLÈVEMENT D'ÉCHANTILLON DE TISSU AVEC INSPECTION VISUELLE DE L'ÉCHANTILLON

(30) Priority: 31.03.2006 US 788567 P
(43) Date of publication of application: 31.12.2008
(60) Divisional application: 16169575.4
(73) Proprietor: Bard Peripheral Vascular, Inc., Tempe, AZ 85281 (US)
(72) Inventor: ANDREASEN, Jørgen, 4700 Næstved (DK); VIDEBÆK, Karsten, 4040 Jyllinge (DK); GUNDBERG, Tomas, 4130 Viby Sjælland (DK); LARSEN, Lars, Erup, 2760 Måløv (DK)
(74) Representative: Tomlinson, Edward James
(86) International application number: PCT/DK2007/000166
(87) International publication number: WO 2007/112751

(56) References cited:
- WO-A-96/24289
- WO-A-2007/021904
- DE-A1- 19 715 284
- US-A- 5 234 000
- US-A- 5 526 822
- US-A- 5 569 284

## Description

### Background of the invention

In modem medicine it is often desirable or necessary to harvest tissue samples from a target tissue region or tissue site inside a human or animal body for the purpose of diagnosing a suspected malignancy.

Minimally invasive methods have partially replaced traditional surgical biopsies to minimize pain, discomfort and morbidity associated with surgical interventions. In the taking of biopsy tissue samples, it is frequently desirable to harvest multiple tissue samples in a single biopsy procedure to ensure that sufficient tissue from the target tissue region is available for analysis. Traditionally, such harvesting of multiple tissue samples required the physician or operator to make multiple passes with a needle-based device to acquire sufficient tissue. To solve this issue, prior art systems primarily adapted for breast biopsy applications provided the operator with the option of harvesting multiple tissue samples in a single device insertion. Such systems considerably ease the task of harvesting a larger number of samples, but they suffer from important limitations.

If it is desired that more than one biopsy sample is to be harvested in a single device insertion, the first tissue sample that is harvested will have to be removed from the sampling components of the biopsy device such as an tissue basket or container before more tissue samples can be harvested. Prior art biopsy devices have chosen between two basic tissue sample collection approaches; a manual and an automatic approach.

The manual systems provide the option of mechanically removing tissue samples from a tissue-receiving component with tweezers or similar means. The manual system enables the operator to visually inspect harvested tissue samples, and to maintain their relative orientation in the target region by placing the harvested tissue samples on strips of paper. This process is relatively slow and relies on human intervention which increases the risk of bio-contamination and other tissue sample degradation and errors.

US patent 5,526,822 discloses an automatic biopsy device capable of harvesting multiple samples from a target region inside a patient body and collect and individually place the harvested tissue samples in a flat substantially rectangular tissue sample cassette.

US patent 6,638,235 discloses another prior art automatic biopsy device.

PCT application wo 96/24289 A describes an arrangement essentially similar to that of US patent 5,526,822 which may include a transparent cassette housing frame for viewing collected specimens.

The automatic biopsy devices are often based on the application of vacuum suction to aspirate tissue samples from the point of sampling or target region into a storage container outside the body of the patient. This method permits the operator to concentrate on the task of harvesting multiple biopsies in a single device insertion without interruptions. However, the tissue sample storage may be more or less random and the storage container or cassette may become clogged, in effect spoiling the function.

### Description of the Invention

It is an object of preferred embodiments of the present invention to at least partially overcome the drawbacks of the prior art devices known to the inventors. One object of preferred embodiments of the invention is to provide a new tissue-ejecting and tissue-collecting system. Another object of preferred embodiments of the invention is to provide a system, which facilitates visual inspection, and which reduces the risk of tissue-sample degradation.

The present invention provides a biopsy device according to claim 1. Preferred aspects of the invention are provided according to the dependent claims.

Embodiments of the invention are described below.

A preferred embodiment of the automated biopsy device may also permit the operator to visually inspect each tissue sample immediately following its excision from the body of the patient without requiring the operator to remove the entire biopsy device from the body of the patient.

The visual inspection system of the present invention is inter alia advantageous where tissue samples are harvested from multiple locations such as in connection with biopsying of the prostate. Under these circumstances it is desirable that all tissue samples are adequate in the sense that they allow a reliable diagnosis to be made. Ensuring that harvested tissue samples are adequate before submitting these for pathologist analysis may reduce the number of patient revisits caused by inadequate biopsy procedures where insufficient or damaged tissue material was produced. Accordingly, the inclusion of suitable visual inspection means on a multiple automated biopsy device has the potential of significantly reducing patient anxiety, patient discomfort and medical costs to the healthcare system.

The inspection system may comprise at least one of: an ocular, a periscopic cone, and a camera system.

An embodiment of an automated biopsy device comprising, e.g. a substantially cylindrical, rotating tissue-collecting cassette with a number of separate tissue-collecting chambers coupled to an ejector system that lift tissue samples out of a tissue-receiving basket or container into the tissue sample cassette while at the same time providing the operator with an active or passive optical inspection means for inspecting each tissue sample has been conceived as further described below.

One advantage of preferred embodiments of the present device over prior art is that it presents a tissue collection cassette with chambers that may be made of a transparent material and are semi-open, to provide easy insertion of one or more tissue samples into one or more cassette chambers while at the same time permitting visual inspection of individual tissue samples immediately following insertion of said tissue samples into said chambers.

According to one embodiment of the invention, the tissue sample cassette may be operatively coupled to a passive or active visual inspection means such as an ocular, a periscopic cone or a camera that may provide enhanced visual inspection of each harvested tissue sample subsequent to its collection. The visual inspection means enables the biopsy device operator to ensure tissue sample adequacy immediately following excision and address potential inadequacies by harvesting one or more additional tissue samples from the target region.

As an additional feature, the visual inspection means may provide a means of magnifying a harvested tissue sample for an enhanced more detailed inspection and evaluation.

The active visual inspection means may comprise a camera such as a digital camera, because digital cameras enable transmission or projection of tissue sample images onto screens for improved visualization of harvested tissue samples.

The tissue sample images may furthermore be transmitted live to one or more remote locations to permit more spectators to participate in a biopsy session, for instance during training sessions, medical conferences or during medical research. According to another embodiment of the present automated biopsy device, the tissue sample cassette incorporates light illuminating means such as one or more light sources selected from the group of LEDs, laser devices, light bulbs etc to illuminate the individually harvested tissue samples after collection to assist the operator during visual inspection of tissue samples.

An advantageous embodiment of the present biopsy device comprises means to support individual automatic and systematic collection of one or more tissue samples when they have been cut from the target region or site in the patient's body and subsequently transported out of the body of the patient. The tissue sample cassette is configured to maintain the axial and radial orientation of the individual tissue samples.

One embodiment of the invention comprises a tissue sample cassette or cassette that is removably attached to a disposable unit of the automated biopsy device and may be removed from the disposable unit following the biopsy procedure. As the harvested tissue samples are still held in their individual cassette compartments, the cassette may function as a tissue sample storage medium. For instance, the cassette may be inserted in a storage container that may be filled with formalin or a similar storage agent, whereupon tissue autolysis is halted and the tissue samples may be sent to the pathologist without further degradation.

Yet another embodiment of the invention comprises a cassette which includes metric marcs, to assist the operator in assessing tissue sample adequacy and the cassette may optionally be configured to additionally include numeric markings on each cassette chamber, to assist the operator and the pathologist in keeping track of harvested tissue samples.

Generally, the sample-collecting device may e.g. comprise a rotatable drum, which comprises the plurality of cavities arranged along its circumference. In the present context, the term "drum" is to be understood as any rotatable element, including a cylindrical or conical element, or any other rotatable element. The outer surface of the drum may be smooth-walled, or it may feature a number of projections or depressions, such as elements for collecting and/or ejecting tissue from the sample-receiving device.

The drum may be rotatable around an axis of rotation, which is essentially parallel to a longitudinal axis of the hollow needle.

The inspection system of preferred embodiments of the present invention may be arranged such that it allows for inspection of a tissue sample after ejection of the tissue sample into one of said cavities and after relative movement of the sample-receiving device and the sample-collecting device to relatively displace said one cavity accommodating the tissue sample away from that position, in which the tissue sample has been ejected into said one cavity. Hence, collecting of a further sample may take place, while the previously collected sample is simultaneously being inspected.

The sample-collecting device is preferably comprised in a disposable unit of the biopsy device. Preferably, the disposable unit is releasably mountable in a reusable unit. Most preferably, the disposable unit includes those elements of the biopsy device, which come into contact with body tissue, body fluid or other body matter, or which otherwise are exposed to contamination. The reusable unit preferably includes driving elements for the transport of the sample-receiving device between the first extended position and the second retracted position, battery cells, operator interface, control circuit, and/or other components which can be kept physically isolated from tissue, body fluid and other body matter, and which consequently may not be exposed to contamination, and which do not come into contact with the patient's tissue or body fluids.

The inspection system may be provided as a part of the disposable unit or as a part of the reusable unit. In case the inspection system includes costly optics and/or camera facilities, it is preferably included in the reusable unit. In some embodiments, the inspection system is only partially included in the biopsy device, with parts of the inspection system being provided remote from the biopsy device. For example, the biopsy device may include a camera and a wired or wireless signal transmission element for communicating an output signal of the cameral to a remote receiver, such as a monitor or a data-processing facility.

It should be understood that the mutual tissue-collecting chamber discussed above is regarded as a separate invention.

In all aspects of the present invention, the ejector system may, in addition to the ejector systems described above, include a setup for ejecting tissue be liquid flushing, e.g. as disclosed in international patent publication No. WO 2006/005345.

In order to move the sample-receiving device, e.g. to move the sample-receiving device in the hollow needle between the first extended and the second retracted positions, there may be provided a transport device comprising a bendable elongate element, e.g. as disclosed in international patent publication No. WO 2006/005344.

To introduce the hollow needle and the sample-receiving device into suspect (target) tissue mass and to sever a tissue sample, there is preferably provided at least one firing mechanism for causing the hollow needle and the sample-receiving device to be longitudinally displaced in a distal direction, so as to penetrate body tissue at or near the suspect tissue mass. The same firing mechanism, or alternatively a further firing mechanism, may be provided for causing the hollow needle to be longitudinally displaced in a distal direction from a first position, in which the sample-receiving device projects from the distal end of the hollow needle, to a second position, in which the hollow needle essentially accommodates the cavity of the sample-receiving device, so as to sever said tissue sample from remaining body tissue at the harvesting site. For example, two user-operable firing mechanisms may be provided as disclosed in WO 2006/005342.

In preferred embodiments of the invention, the sample-receiving device and the hollow needle are comprised in a disposable unit, which is releasably attachable to a reusable unit including e.g. drive components for providing a driving force for transporting the sample-receiving device. In such embodiments, there is preferably provided a control system for controlling movement of the transport device and for arresting the sample-receiving device in the second retracted position, as disclosed in international patent publication No. WO 2006/005343. For example, the control system may be configured to automatically detect a distance between the first extended position and the second retracted position of the sample-receiving device, as disclosed in WO 2006/005343.

### Detailed description of embodiments of the invention

Embodiments of the invention will now be described further with reference to the accompanying drawings, in which:
Figs. 1-15 illustrate a -tissue-collecting device and ejector system;
Figs. 16 and 17 generally illustrate an embodiment of a biopsy device according to the invention;
Figs. 18-22 illustrate an embodiment of a transport mechanism and cutting mechanism.

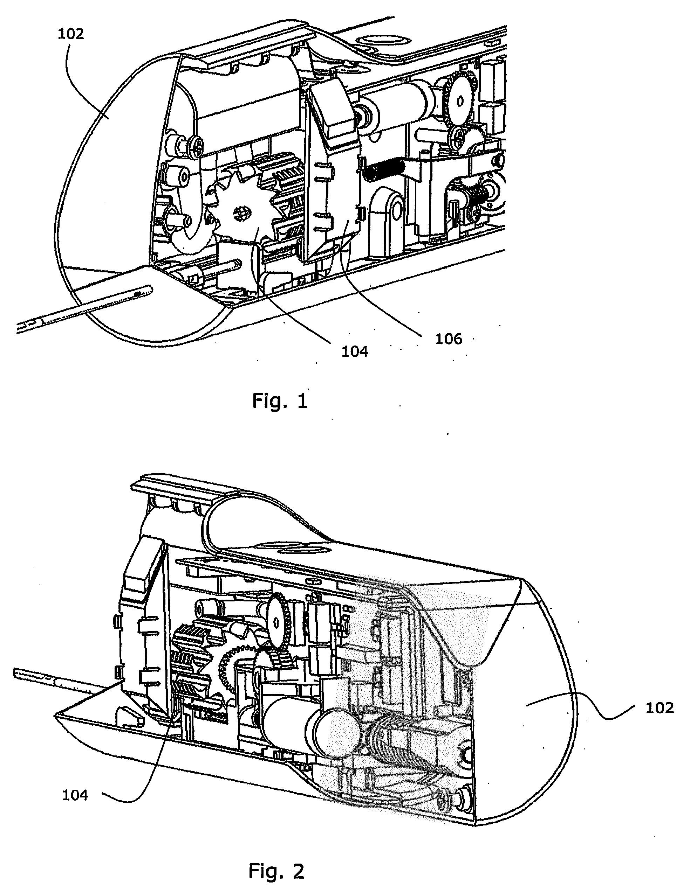

Figs. 1-3 show three 3D views of an embodiment of the biopsy device with the tissue sample cassette system and an optical cone placed adjacent to the cassette.

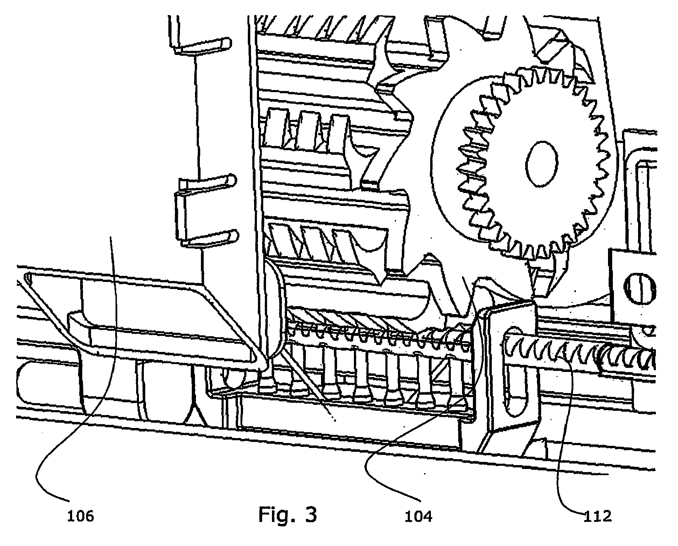

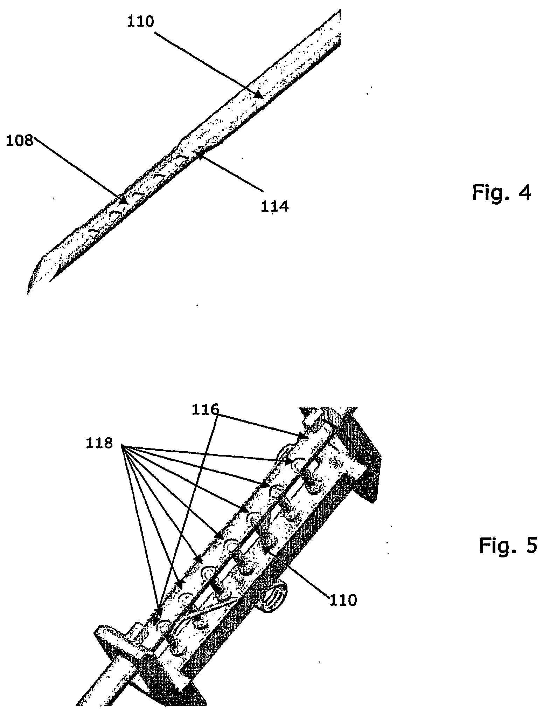

Fig. 1 is a semi-frontal view where a housing 102 of the biopsy device has been made transparent from the left side, showing a tissue-collecting cassette 104 behind an ocular 106. Fig. 2 is a semi-rearward view from the left, showing more clearly the shape of the cassette 104. Fig. 3 is a detail semi-rearward view of the cassette 104 from the left. The ejection zone where the cassette 104 meets a tissue-receiving basket or container and the placement of the ocular 106 relative to the cassette 104 are shown. Optical inspection of the tissue-sample takes place immediately following ejection of the tissue sample from the tissue-receiving container 108 (cf. Fig. 4).

One embodiment of the present biopsy device comprises a disposable unit comprising invasive components, and a non-disposable unit comprising the driver components. The biopsy device works by positioning a generally tubular tissue-receiving container 108 in a suspect tissue region. The tissue-receiving container 108 is movable between a first advanced and a second retracted position within the inner lumen of a retractable cutting cannula 110, cf. Fig. 4. The cutting cannula 110 and the tissue-receiving container 108 are placed in their first advanced positions during insertion into the anatomy of the patient.

Subsequent retraction of the cutting cannula 110 exposes a lateral opening in the tissue-receiving container that leads a cavity in the tissue-receiving container 108, and permits adjacent tissue to prolapse into the cavity of the tissue-receiving container. Vacuum, drawn through the inner lumen of the cutting cannula 110, further assists the prolapse of tissue. In the retracted position of the cutting cannula 110 where the lateral opening of the tissue-receiving container 108 is fully exposed and the cavity has been filled with tissue from the target region, the cutting cannula 110 is rapidly advanced and the prolapsed tissue is severed and captured, as the end portion of the cutting cannula forms a circumferential cutting edge. During a subsequent retraction of the tissue-receiving container 108 towards its second retracted position by means of a transport mechanism, the harvested tissue sample is moved out of the body of the patient.

In one preferred embodiment the transport mechanism is configured as a bendable toothed rack 112 (cf. Fig. 3) that interfaces with a number of cogs to provide linear motion of the tissue-receiving container 108. It is, however, to be understood that other flexible rods, racks or bands may substitute the toothed rack 112. When the tissue sample has been moved fully out of the body of the patient, it may be ejected from the cavity of the tissue-receiving container 108 and collected in the tissue-collecting cassette 104. The tissue-receiving container 108 may then be repositioned in the suspect tissue region by means of the toothed rack 112, and a new tissue sample may be harvested by repeating the procedure as outlined above.

As shown in Fig. 4, the present embodiment of the biopsy device comprises an outer cutting cannula 110 (or hollow needle) with a proximal end and a distal, sharpened end 114, including the circumferential cutting edge, as well as the flexible toothed rack 112 (cf. Fig. 3) that is received in the inner lumen of the cutting cannula and is longitudinally movable along the axis of the outer cutting cannula between a first advanced and a second retracted position. The tissue-receiving container 108 in Fig. 4 is herein also referred to as a sample-receiving device.

The toothed rack 112 is operatively connected to the hollow tubular tissue-receiving container 108 comprising a container cavity and an elongate opening in the topmost part. Said container is arranged in continuation of the distal end of the toothed rack 112.

Referring to Fig. 5, an ejector opening 116 in the proximal part of the outer cutting cannula 110 has a length that corresponds with the length of the opening in the tissue-receiving container 108 and permits access to the container cavity. The second retracted position of the tissue-receiving container corresponds with this opening, and the tissue sample may thus be collected from the cavity of the tissue-receiving container 108 through the ejector opening 116. In the bottom portion of the cutting cannula 110, and directly opposite the ejector opening 116, an axially aligned row of ejector pin holes 118 is provided. These holes correspond with an equal number of axially aligned ejector pin holes in the bottom of the tissue-receiving container 108, and when the tissue-receiving container is in its second retracted position, the two sets of holes are vertically aligned.

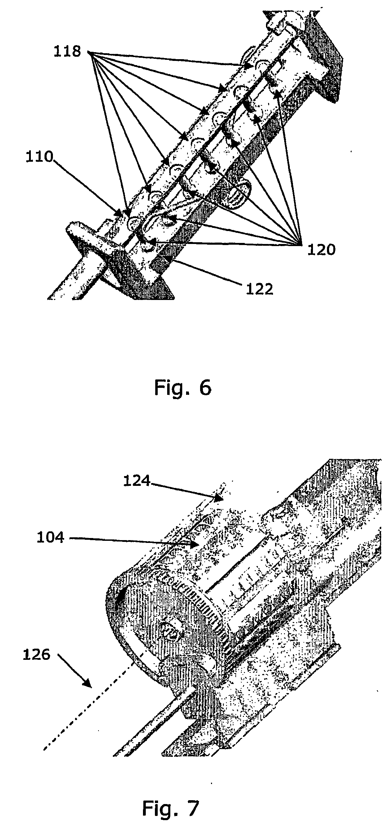

As shown in Fig. 6, the ejector pin holes in the cutting cannula 110 and the tissue-receiving container 108 are configured to receive a number of vertically movable ejector pins 120 that are mounted on an ejector frame 122 and are introduced into the tissue-receiving container 108 from a point below the bottom of the cutting cannula 110. The ejector pins 120 push tissue kept in the container through both the elongate opening of the tissue-receiving container and the ejector opening in the cutting cannula. When tissue has been pushed all the way through the ejector opening, it may be collected by a suitable means of collection.

As shown in Fig. 7, the cylindrical, elongate tissue sample cassette (rotatable drum) 104 comprises such a means of collection. It is removably placed in a cassette housing 124 that is removably attached to a disposable part of the biopsy device, and is stepwise rotatable about a central axis 126 that is parallel to the axis of the outer cutting cannula 110.

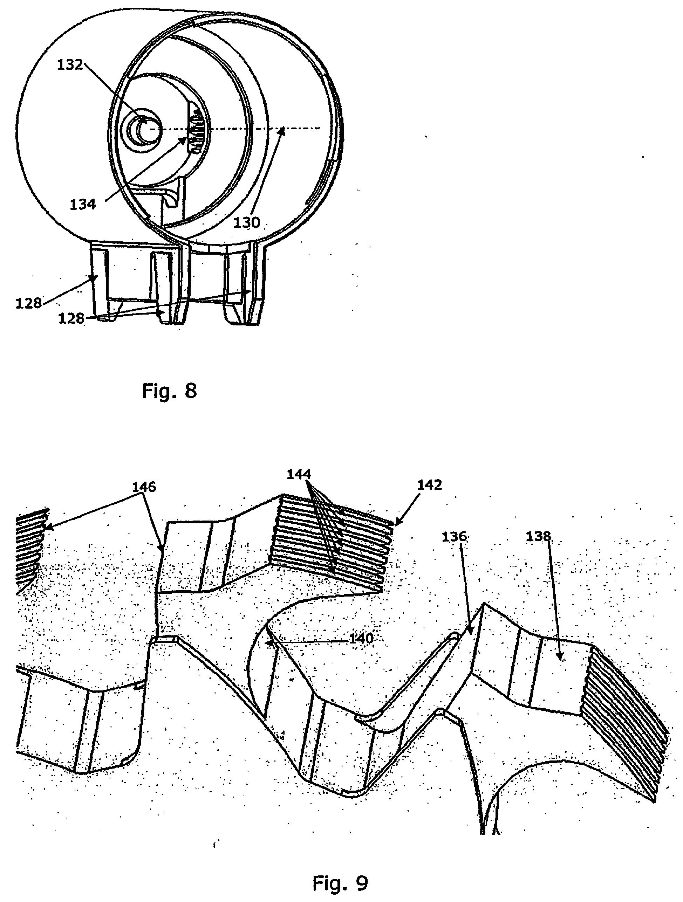

As shown in Fig. 8, the cassette housing is removably attached to the disposable unit by means of four snap locks 128. Said housing has a central axis 130 defining the centre of rotation of the cassette 104, and hence co-inciding with central axis 126 (cf. Fig. 7). A central axle 132, aligned along the central axis 130, may provide support for the cassette 104, but cassette designs eliminating the central axle are also encompassed by this invention. In one embodiment, a short central axle is received in a central hole in the cassette and provides support to the proximal end of the cassette, while a central snap-lock provides support for the distal end. A worm gear 134 is provided for rotating the cassette 104.

The cassette 104 has one or more tissue-collecting chambers (or tissue-retaining cavities). The chambers comprise axially aligned elongate cavities that are sunk into the outer periphery of the cylindrical cassette 104, perpendicular to the direction of rotation. The number of chambers is assumed to be between 5 and 15, but may be lower or higher. Each chamber has the following features, cf. Fig. 9:
1. A flat face 136 that is roughly perpendicular to the outer periphery.
2. Adjacent to the face 136 a small cavity 138 is provided that fits the outer contour of the cutting cannula 110. In the default position of the cassette 104, the cavity 138 is positioned above the cutting cannula 110 to support the toothed rack 112 to prevent buckling-out of the rack.
3. Opposite from and at a distance to the face 136 a second face 140 is provided that is shaped as a semi-circle. The semicircular shape of this face creates a cavity where a tissue sample may rest after having been lifted out of the tissue- receiving container 108, as will be described below.
4. The intersection of the semicircular face with the outer periphery of the cassette comprises a wedge-shaped lifting edge 142.
5. In the outer periphery of the cassette, a number of evenly spaced grooves and lands 144 circle the entire cassette parallel to the plane of rotation. The lands are evenly spaced and are configured to mesh with the ejector pins 120 as a part of the ejection sequence. The pins 120 are configured to slide in the grooves when the cassette 104 rotates, with the aim to permit the cassette 104 to rotate while the pins 120 are fully extended. These grooves and lands impact on the shape of the lifting edge, imparting on it a shape like a fork with very broad and short teeth.
6. The gap between the flat face and the edge of the semicircular face comprises the opening 146 of each chamber, through which tissue will enter the chamber. The chambers are dimensioned to easily receive the biggest sample that may be produced by a tissue-receiving container of a given size.

The nature and configuration of these features is illustrated in Fig. 9.

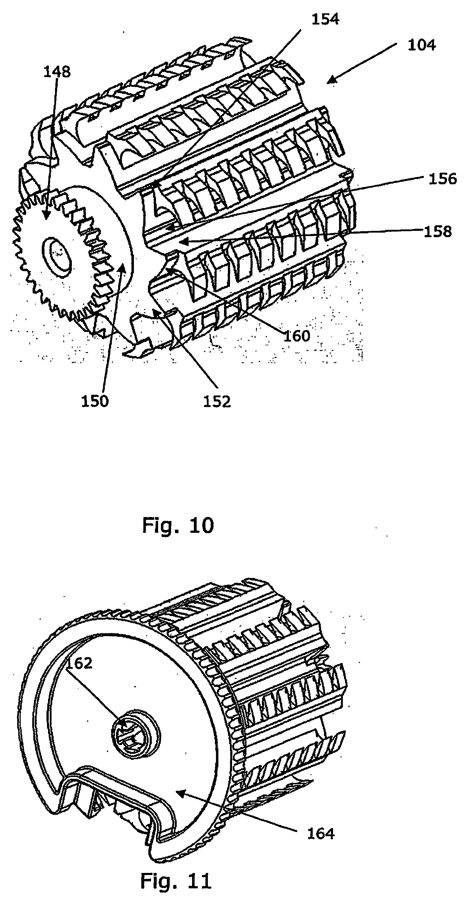

In addition to these features, the cassette cylinder has a flattened proximal end and a flattened distal end that are parallel to the plane of rotation. These ends are discussed further below with reference to Fig. 10.

Referring to Fig. 10, the proximal end provides a mechanical interface that may be in operative connection with a power transmission mechanism to allow the stepwise rotation of the cassette about its central axis. The direction of rotation is clockwise, when the cassette is viewed from the proximal end towards the distal end.

In one embodiment, the mechanical interface to the power transmission mechanism is a gear wheel 148 that is placed parallel to the plane of rotation of the cassette 104. In addition, the gearwheel is connected to the body of the cassette 104 with a spacer bushing 150. In the center of the gear wheel and the spacer bushing 150 may be provided a hole that may be configured to receive a central axle.

Further, both the proximal and the distal end of the cassette provide means of moving the ejector frame in a vertically reciprocating motion to alternately insert the ejector pins 120 into the cavity of the tissue-receiving container 104 to eject a tissue tissue sample and retract them from said cavity when a tissue sample has been ejected. In one embodiment, the means of moving the ejector frame are twin actuator ridges 152 roughly resembling the blade of a circular saw that are mounted on the proximal and distal ends of the cassette body parallel to the plane of rotation. The actuator ridges 152 comprise ridge tops 154, ridge flanks 158, ridge bottoms 156, and ridge ramps 160.

In one embodiment the reciprocating motion of the ejector frame is stepwise to allow time for the device to carry out other functions. Such stepwise motion may be accomplished through suitable design of the actuator ridges and/or through alternate pausing and starting of the rotation of the cassette.

As shown in Fig. 11, the distal end of the cassette features a central snap-lock 162 that is rotatably received in a central hole in a lid-like portion 164 of the cassette housing and acts as a cassette support axle. Said lid-like portion is configured to be removed from the cassette housing following a biopsy procedure to provide access to the tissue samples. The snap-lock ensures that the cassette stays attached to the lid-like portion during removal and further acts as a gripping surface permitting the operator to handle the cassette without touching the samples. In addition, the snap-lock permits the cassette to be removed from the lid-like portion if the operator desires to separate these two items.

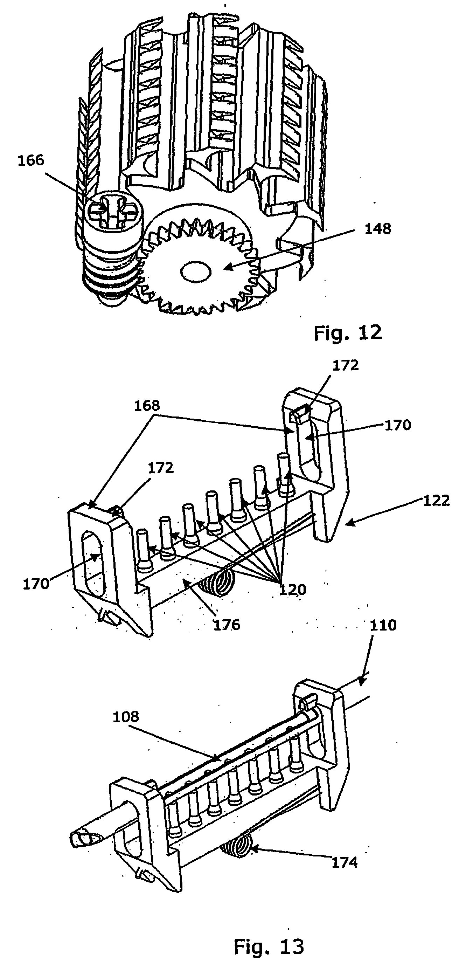

Rotation of the cassette is driven by a motor that is controlled to provide step-wise rotation at predetermined intervals. This drive system will be described further below with reference to Fig. 12.

Referring to Fig. 12, the gear wheel of the cassette is in operative connection with a worm gear 166 that is vertically mounted in the housing of the cassette and has an upper and a lower end. The upper end comprises a screw slot with a cross-point configuration that may receive a motor axle in operative engagement with the cassette motor. The lower end is rotatably lodged in a recess in the housing of the cassette.

To provide the operator of the device with the option of harvesting multiple samples in a single device insertion, samples that are held in the tissue-receiving container following a biopsy will have to be removed from said container. Removal is accomplished by means of a vertically movable ejector frame that is in operative engagement with the cassette, cf. the below description of Fig. 13.

As shown in Fig. 13, the ejector frame 122 comprises two vertical arms 168, each with a motion guide slot 170 that is shaped to receive the outer cutting cannula 110. Each arm 168 further comprises an actuator pin 172 that is configured to interface with one of the actuator ridges of the cassette 104. Thus, rotation of the cassette 104 may be translated to vertical motion of the ejector frame 122 between a lower default position and an upper elevated position. Motion from the lower default position to the upper elevated position is driven by a wire spring 174 that is placed below the ejector frame 122, while motion from the upper elevated position to the lower default position is driven by the teeth of the actuator ridges pressing down on the actuator pins 172.

Between the two arms 168 is placed a middle section 176 with a length that is slightly larger than the length of the cylinder and which comprises a number of vertical ejector pins 120. Each of these ejector pins is configured so as to fit in a corresponding ejector pin hole in the bottom of the cutting cannula 110, and a corresponding ejector pin hole in the bottom of the tissue-receiving container 108.

To provide the operator with the option of visually inspecting a harvested tissue sample following ejection without having to interrupt the biopsy procedure to remove the sample from the biopsy device, an ocular, a periscopic cone, a camera or another means of gathering an image of the tissue sample from one location and projecting it to another location immediately following the placement of the tissue-sample in the tissue-collecting cassette, is placed in visually unobstructed communication with the cassette.

In one embodiment of the device, a periscopic cone that is made of acryl or a material with similar light-conducting properties is placed immediately adjacent to the point where the cassette meets the tissue-receiving container to collect a tissue sample. Thus, the tissue sample may be visually inspected immediately after being ejected from the tissue-receiving container and the operator may inspect the harvested tissue sample to assess its adequacy.

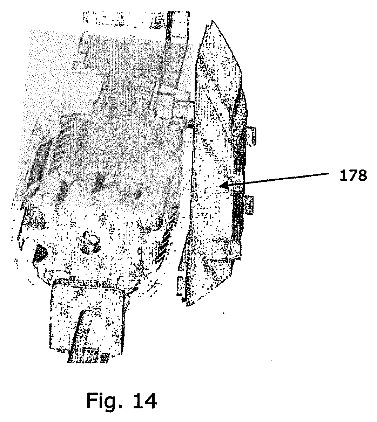

The periscopic cone 178 illustrated in Fig. 14 preferably comprises a vertical wand of acryl or a similar optically conductive material that constitutes an optical connection between a glass plate placed immediately adjacent to the bottom of the cassette and an ocular placed in a device shell that constitutes the outer surface of the biopsy device. The wand has a width that is substantially equal to the depth of the cassette and features a mirror-like surface immediately adjacent to the glass plate and angled relative to this glass plate in a 45-degree angle. Said mirror-like surface is responsible for projecting the image from the glass plate to the ocular.

The ocular may or may not comprise means of magnifying the tissue sample to provide the operator with enhanced visualization. Such means may constitute a convex lens, an arcuate surface or a similar means of magnification. The cassette may or may not comprise a light source to provide the operator with enhanced visualization.

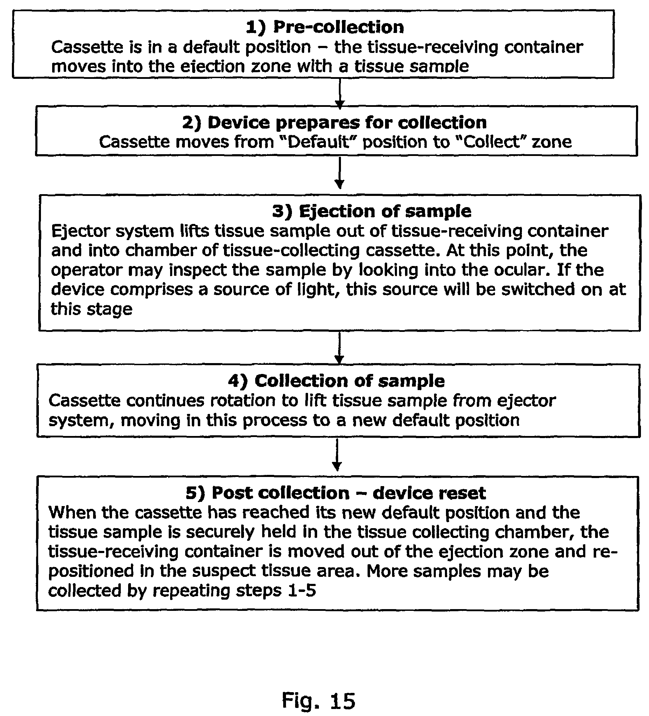

The generic operation sequence of the above described tissue sample cassette is depicted in Fig. 15. A more thorough description of the operation sequence is provided below.

An operation sequence of the embodiment of Figs. 1-15 is described below:

### 1. Pre-collection

Prior to a biopsy sequence, the device is assembled and the invasive components (cutting cannula and tissue-receiving container) are inserted into the body of the patient and positioned at the target region inside the patient's body.

In a biopsy sequence, a tissue sample is harvested at the target region, whereupon it is held in the tissue-receiving container. The toothed rack is subsequently engaged to transport the tissue-receiving container containing the sample from this point to the point of collection - corresponding with the position of the tissue-collecting cassette. During transport of the tissue sample, the cassette is held in a default position. When the cassette is in such a default position, it is positioned in such a way that the cavity adjacent to the flat face of a given tissue-collecting chamber partially covers the ejector opening in the outer cutting cannula. This is done to prevent the toothed rack from buckling during advancement of the tissue-receiving container.

Further, the cassette is vertically aligned with the second retracted position of the tissue-receiving container in such a way that the circular path of a given lifting edge intercepts a point just above the edges of the lateral opening of the cutting cannula during rotation of the cassette. Thus, a given lifting edge may partially enter the lumen of the cutting cannula and the cavity of the tissue-receiving container at predetermined intervals when the cassette is rotated. Said intervals are variable according to the distance between tissue-collecting chambers and to the speed of rotation of the cassette. For safety purposes, the biopsy device is constructed in such a way that the cassette may only start rotating when the tissue-receiving container has been moved to its second retracted position.

In the default position, no part of the cassette enters the lumen of the cutting cannula or the cavity of the tissue-receiving container, and both cutting cannula and tissue-receiving container are free to move. In this way, the tissue-receiving container may be moved between its first advanced and its second retracted position without interference from the cassette.

### 2. Device prepares for tissue sample collection

Following proper positioning of the tissue-receiving container at the point of collection, the worm gear may be engaged by the handle unit to produce rotational motion of the gear wheel and thus the cassette. In rotation, the cavity adjacent to the flat face of a given chamber is moved away from its position immediately above the tissue-receiving container. This causes the gap comprising the opening of a given tissue-collecting chamber to present itself in a position immediately above the tissue-receiving container to receive a tissue sample.

### 3. Ejection of tissue sample

One or more ejector pin holes are placed in the bottom of the tissue-receiving container. These holes match a similar number of holes in the cutting cannula and when the tissue-receiving container has been correctly positioned in its second retracted position, the holes in the tissue-receiving container are aligned with the holes in the cutting cannula.

The ejector pin holes are configured to receive one or more ejector pins that are mounted on an ejector pin frame and are vertically movable between a lower default position and an upper extended position.

When in their lower default position, the ejector pins are completely retracted from the holes in the tissue-receiving container and the cutting cannula. When in their upper extended positions, the pins are fully inserted into the holes and protrude from these to a point slightly above the top of the cavity of the tissue-receiving container.

During their movement from the lower to the upper position, the ejector pins will engage the tissue sample and lift it out of the tissue-receiving container to position the tissue sample in a given gap comprising the opening of a given tissue-collecting chamber. At the top of the lifting motion, the sample is fully inserted in the gap, and the ejector pins extend to a point above the outer periphery of the cutting cannula.

The lifting and lowering motion of the ejector pins is controlled by the interplay of the cassette actuator ridges and the actuator pins of the ejector frame. When the cassette is in a default position, the actuator pins rest on the tops of the tips of a given pair of actuator ridges. When the cassette starts rotating, the actuator pins slide off the tops and start moving up the adjacent sides, and the wire spring that is positioned below the ejector frame exerts upwards pressure on the frame. Thus, the ejector frame is moved from its lower default position towards its upper extended position, intercepting and lifting the tissue sample in this movement. The speed of the upwards motion may be controlled by the speed of rotation of the cassette and/or the steepness of the ridge flanks of the actuator ridges.

As the cassette rotates, the tissue sample is moved into the line of sight of the periscopic cone. In one preferred embodiment, an LED or similar means of illumination is turned on to clearly illuminate the tissue sample. The light is kept on for a period of time sufficient to enable the operator to fully inspect the harvested tissue sample, whereupon it is turned off to save energy.

### 4. Collection of sample

When the movement of the ejector frame from the lower default position to the upper extended position has been carried out, the tissue sample is positioned in the opening gap of a tissue-collecting chamber. As will be recalled, the path of the lifting edge intercepts a point just above the edges of the lateral opening of the cutting cannula, and thus below the tissue sample when it has been maximally elevated by the ejector pins. As the rotation of the cassette continues, the grooves in the surface of the cassette mesh with the ejector pins, permitting the lifting edge to continue its movement while the ejector pins are still extended. In this way, the lifting edge may move to a point beyond the tissue sample before the top of the wedge-shaped lifting edge makes contact with the sample and lifts it off the ejector pins. Thus, the tissue sample is securely positioned in a tissue-collecting chamber.

### 5. Post collection

When the tissue sample has been lifted off the ejector pins, the cassette continues its rotation until the next chamber has been placed in its default position. During this rotation, the ejector pins are moved to their lower default position by the interplay of the actuator pins with the ridge ramps of the actuator ridges, and the ejector pins are moved out of the holes in the tissue-receiving container and the cutting cannula. When the ejector pins have moved fully out of the holes and the cassette has been positioned in a default position, the tissue-receiving container may be moved to its first advanced position, and another sampling sequence may be initiated.

It has been found that the sample-collecting device (i.e. cassette) and the ejector system of Figs. 1-15 are best suitable for certain types of tissue. Generally, the setup of Figs. 1-15 is suitable for relatively firm tissue, such as cartilage and muscle tissue.

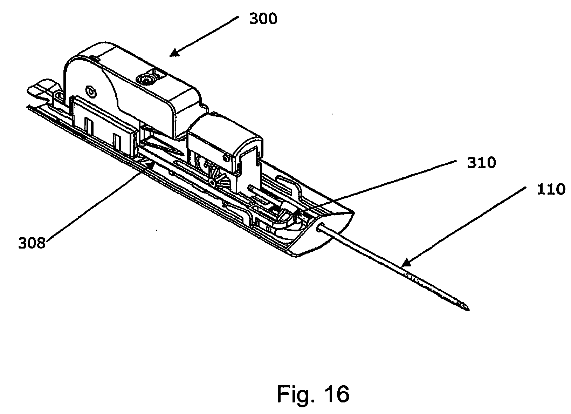

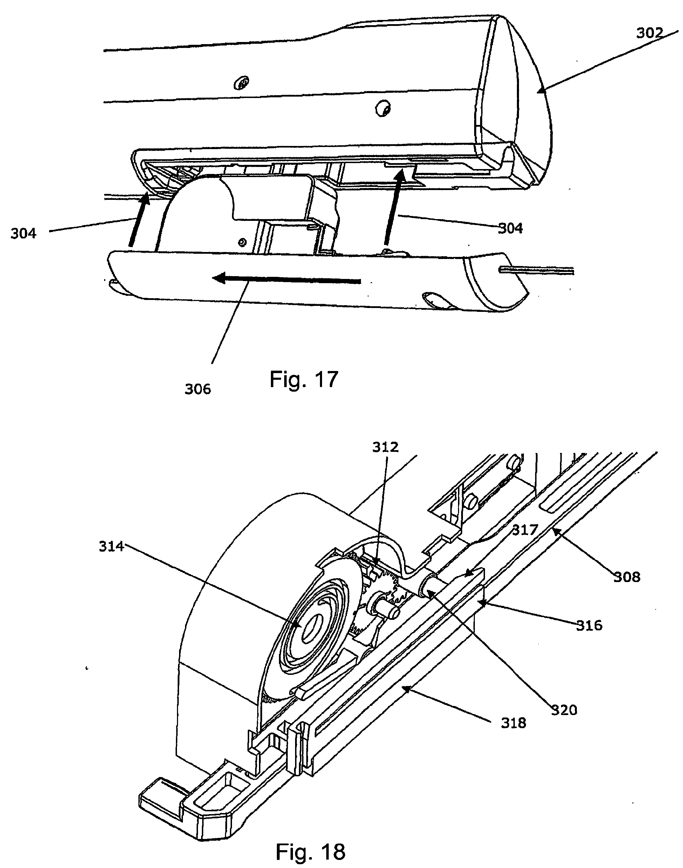

Figs. 16 and 17 generally illustrate an embodiment of a biopsy device according to the present invention. The device comprises a disposable unit 300 (Fig. 16), which is insertable into a reusable handle unit 302 (Fig. 17). The disposable unit 300 comprises those components which come into contact with tissue and other body matter of the patient, whereas the handle unit comprises other components, such as control circuit and motor, which do not come into contact with body matter. The disposable unit 300 is insertable into the handle unit as indicated by arrows 304 and 306 in Fig. 17. The disposable unit comprises the outer cutting cannula 110 (cf. Fig. 16) as well as the inner needle, also referred to as sample-receiving device with the tissue-receiving container 108 (not visible in Fig. 16). A needle driver 308 is provided, which is non-detachably secured to the cutting cannula 110, e.g. via an attachment element 310.

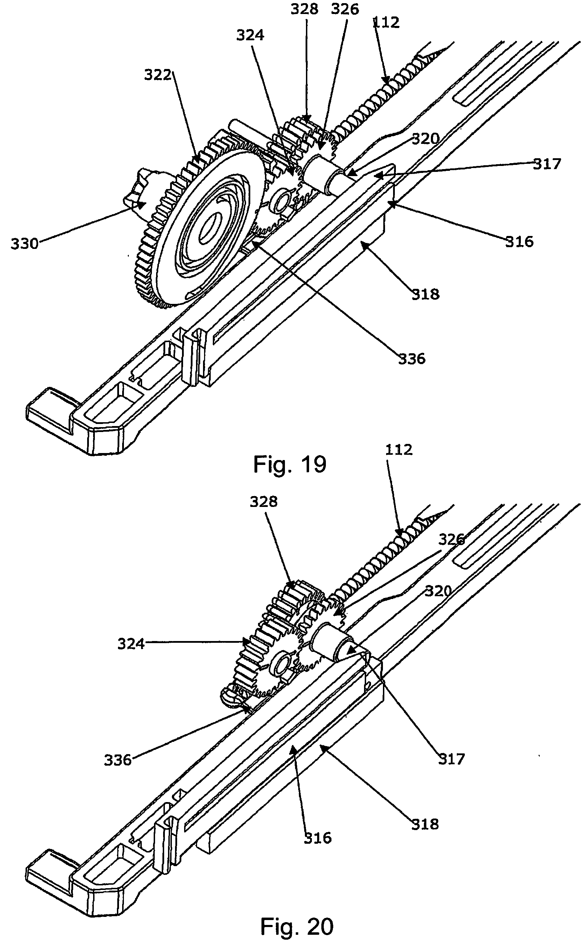

Figs. 18-22 generally illustrate an embodiment of a transport mechanism and cutting mechanism incorporated in the disposable unit 300. There is provided a drive 312 for engaging the toothed rack (elongate flexible transport element) 112, cf. e.g. Figs. 3 and 19. A coiling device 314 is provided for coiling up the toothed rack 112 when the sample-receiving device is moved towards its second retracted position. There is further provided an engagement slider 316 having a sloping portion 317, an engagement lift element 318, and a spring-biased connector 320. The function of these elements will be described below.

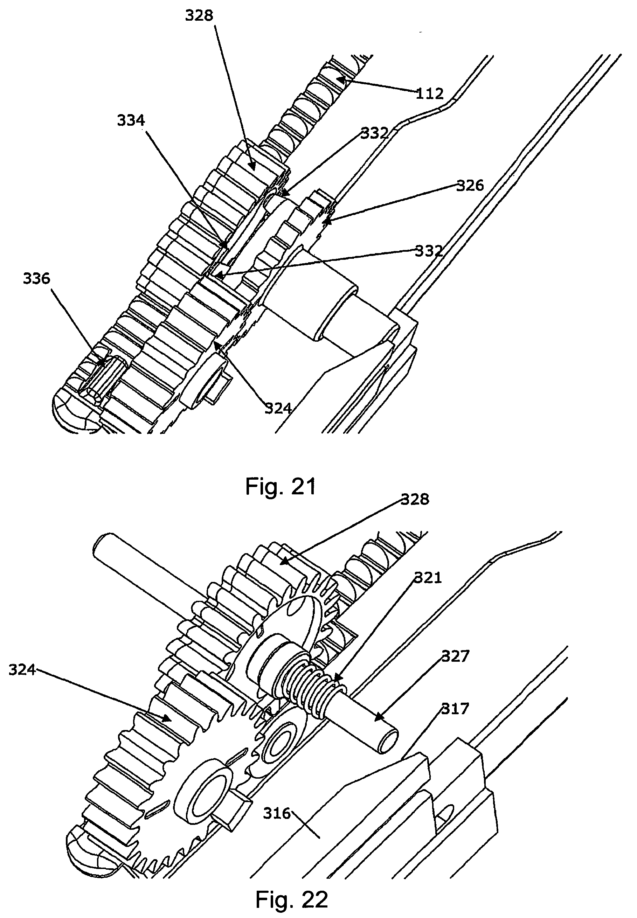

As shown in Fig. 19, the drive 312 includes four gearwheels, 322, 324, 326, and 328. A first gearwheel 322 is formed on an outer circumference of the coiling device 314, and is drivable via a connector shaft 330 for engaging a motor drive of the re-usable unit 302 (cf. Fig. 17). The first gearwheel 322 drives a second gearwheel 324, which in turn drives a third gearwheel 326, which is mounted on a common axle with a fourth gearwheel 328. In the configuration shown in Fig. 19, a side surface of the third gearwheel 326 abuts and engages a side surface of the fourth gearwheel 328. The third gearwheel 326 is transversely slidable out of engagement with the fourth gearwheel 328, cf. Fig. 21, in which the third and fourth gearwheels 326 and 328 are out of mutual engagement. As shown in Fig. 21, engagement elements 332 are provided on the third gearwheel 326 to engage corresponding engagement cavities 334 in a side surface of the fourth gearwheel 328. In both positions of the third gearwheel 326, i.e. in the position of Fig. 19 and the position of Fig. 21, its circumferential gear portion engages the second gearwheel 324. However, in the position of the gearwheels of Fig. 19, a driving force is transmittable from the connector shaft 330 to the toothed rack 112, whereas in the position of Fig. 21, the third gearwheel 326 is idling, and no driving force is transmittable to the toothed rack via the gear system described above.

The third gearwheel 326 is brought into and out of its engagement with the fourth gearwheel 328 by longitudinal displacement of the engagement slider 316 as illustrated in Figs. 19 and 20. In the advanced position of the engagement slider 316 shown in Fig. 19, the spring-biased connector 320 rests against a non-inclined surface portion of the engagement slider 316. As the engagement slider is retracted to the position shown in Fig. 20, the spring-biased connector 320 runs down the sloping surface portion 317. The connector 320 is secured to the third gearwheel, and hence an outward movement of the connector 320 also moves the third gearwheel 326 in an outward direction, i.e. out of engagement with the fourth gearwheel 328, cf. Fig. 21. The third gearwheel 326 and connector 320 are removed from the illustration of Fig. 22 to reveal a spring 321 providing the spring-biasing force to the connector 320. Fig. 22 also illustrates a mounting axle 327 for the third and fourth gearwheels 326 and 328.

An engagement hook 336 is further provided, cf. Figs. 19, 20 and 21. The engagement hook 336 is movable between two positions. In a first position, the engagement hook is in a lowered position, in which it does not engage the toothed rack 112. The engagement hook 336 is coupled to the engagement slider 316 and the engagement lift element 318 in such a way that in the advanced position of the engagement slider shown in Figs. 18 and 19, the engagement hook is in its first position. In this position of the engagement slider 316, the third gearwheel 326 engages the fourth gearwheel 328. Consequently, movement of the toothed rack 112 and hence also of the sample-receiving device with the tissue-receiving container 108 is controlled by the drive 312, including the gearwheels 322, 324, 326 and 328. When the engagement slider 316 is retracted to the position shown in Figs. 20 and 21, the third gearwheel 326 is brought out of engagement with the fourth gearwheel 328. At the same time, due to the coupling of the hook 336 with the engagement slider 316 and the engagement lift element 318, the hook raises and engages an eye provided in a distal end portion of the toothed rack 112, as shown in Fig. 21. As the third gearwheel 326 is brought out of engagement wit the fourth gearwheel 328, movement of the toothed rack and hence also of the sample-receiving device with the tissue-receiving container 108 is no longer controlled by the drive, but by the needle driver 308, which supports the engagement hook 336.

It will hence be appreciated that in the position of Figs. 20 and 21, longitudinal displacement of the needle driver 308 causes the outer cutting cannula as well as the inner needle to displace longitudinally, as the needle driver 308 is secured to the outer needle 110 (cf. attachment element 310 of Fig. 16), and as the needle driver 308 is connected to the toothed rack 112 via the engagement hook 336. The needle driver 308 is connected to a firing mechanism including one or more springs for firing the needle driver in a forward direction to cause the outer cutting cannula as well as the inner needle (with sample-receiving device) to simultaneously advance rapidly. Such simultaneous firing of the outer and inner needles may be used to pierce a target tissue, i.e. to introduce the outer and inner needles into the target tissue mass prior to tissue harvesting.

In the position of Figs. 18 and 19, firing of the needle driver 308 merely causes firing of the outer cutting cannula 310, as the hook 336 and thus also the needle driver 308 are disengaged from the toothed rack 112 and hence from the sample-receiving device and inner needle. Accordingly, when the needle driver 308 is fired in the position of Figs. 18 and 19, the outer cutting cannula 110 may sever tissue drawn into the tissue-receiving container 108 by vacuum suction. A driving force subsequently is applied to the connector shaft 330 is transmitted via gearwheels 322, 324, 326 and 328 to the toothed rack 112, as the third and fourth gearwheels 326 and 328 are in mutual engagement as described above. Hence, the sample-receiving device may be moved between its first advanced position, in which tissue is severed, and the second retracted position, in which a tissue sample is ejected from the tissue-receiving container 108, e.g. by means of the ejector and collecting system described above with reference to Figs. 1-15.

In order to fire the outer cannula 110 and possibly also the inner needle, a spring-loaded firing system may be provided. The firing system may e.g. comprise one single spring or two separate springs, e.g as disclosed in WO 2006/005342.

## Claims

1. A biopsy device configured to harvest at least one tissue sample from a body of a living being, comprising:
a hollow needle (110) with a distal end portion adapted to be introduced into the body;
a cutting mechanism for severing the at least one tissue sample;
a sample-receiving device (108) for receiving the at least one severed tissue sample, the sample-receiving device being receivable in the hollow needle and movable therein between a first extended position and a second retracted position;
a tissue sample-collecting device (104) comprising one or more tissue-retaining cavities;
an ejector system for ejecting the tissue sample from the tissue sample-receiving device into one of said tissue-retaining cavities, when the sample-receiving device is in the second retracted position;
an inspection system (178) allowing an operator of the biopsy device to visually inspect the tissue sample ejected into said tissue-retaining cavity, while said hollow needle remains in the body,
**characterized in that** the ejector system comprises a plurality of movable pins (120) arranged with mutual spacings along a longitudinal axis of the hollow needle, and wherein the hollow needle comprises a plurality of first openings (118) facing said pins, and wherein the sample-receiving device comprises a plurality of second openings, which are aligned with said first openings, when the sample-receiving device is in the second retracted position, so that said pins can penetrate said first and second openings to eject the tissue sample.

2. A biopsy device according to claim 1, wherein the inspection system (178) comprises at least one of: an ocular, a periscopic cone, and a camera system.

3. A biopsy device according to claim 1, comprising a plurality of tissue-retaining cavities for receiving individual tissue samples, and wherein the tissue sample-collecting device and the sample-receiving device are movable relative to each other in such a way that different tissue samples harvested at different times can be accommodated in separate ones of said plurality of cavities.

4. A biopsy system according to claim 3, wherein the sample-collecting device comprises a rotatable drum, which comprises said plurality of cavities arranged along its circumference.

5. A biopsy device according to claim 3, in which the inspection system is arranged such that it allows for inspection of a tissue sample after ejection of the tissue sample into one of said cavities and after relative movement of the sample-receiving device and the sample- collecting device to relatively displace said one cavity accommodating the tissue sample away from that position, in which the tissue sample has been ejected into said one cavity.

6. A biopsy device according to claim 1, wherein the sample-collecting device is comprised in a disposable unit of the biopsy device.

7. A biopsy device according to claim 4, wherein said drum is rotatable around an axis of rotation, which is essentially parallel to a longitudinal axis of the hollow needle.

8. A biopsy device according to any of the preceding claims, wherein said tissue sample- collecting device comprises at least one scooping element (142) arranged on its outer circumference, said scooping element being arranged to collect tissue in the sample-receiving device, when the sample-receiving device is in its second retracted position.

9. A biopsy device according to claim 8, wherein said tissue sample-collecting device is movable between a first elevated position, in which said scooping element does not engage a tissue-receiving container of the sample-receiving device, and a second lowered position, in which said scooping element engages said tissue-receiving container.

10. A biopsy device according to claim 8 or 9, wherein said at least one scooping element forms at least a wall of at least one said tissue-retaining cavities.

## Patentansprüche

1. Biopsievorrichtung, die dazu konfiguriert ist, mindestens eine Gewebeprobe aus einem Körper eines Lebewesens zu gewinnen, umfassend:
eine Hohlnadel (110) mit einem distalen Endabschnitt, der geeignet ist, in den Körper eingeführt zu werden,
einen Schneidmechanismus zum Abtrennen der mindestens einen Gewebeprobe,
eine Probenaufnahmevorrichtung (108) zur Aufnahme der mindestens einen abgetrennten Gewebeprobe, wobei die Probenaufnahmevorrichtung in der Hohlnadel aufgenommen und darin zwischen einer ersten ausgefahrenen Position und einer zweiten eingezogenen Position bewegt werden kann,
eine Gewebeprobensammelvorrichtung (104), die einen oder mehrere Gewebehaltehohlräume umfasst,
ein Ausstoßersystem zum Ausstoßen der Gewebeprobe aus der Gewebeprobenaufnahmevorrichtung in einen der Gewebehaltehohlräume, wenn die Probenaufnahmevorrichtung in der zweiten eingezogenen Position ist, ein Inspektionssystem (178), das es einer Bedienperson der Biopsievorrichtung gestattet, die in den Gewebehaltehohlraum ausgestoßene Gewebeprobe visuell zu inspizieren, während die Hohlnadel im Körper bleibt,
**dadurch gekennzeichnet, dass** das Ausstoßersystem eine Vielzahl von beweglichen Stiften (120) umfasst, die mit gegenseitigem Abstand entlang einer Längsachse der Hohlnadel angeordnet sind, und wobei die Hohlnadel eine Vielzahl von ersten Öffnungen (118) umfasst, die den Stiften zugewandt sind, und wobei die Probenaufnahmevorrichtung eine Vielzahl von zweiten Öffnungen umfasst, die auf die ersten Öffnungen ausgerichtet sind, wenn die Probenaufnahmevorrichtung in der zweiten, eingezogenen Position ist, so dass die Stifte zum Ausstoßen der Gewebeprobe durch die ersten und zweiten Öffnungen dringen können.

2. Biopsievorrichtung nach Anspruch 1, wobei das Inspektionssystem (178) ein Okular und/oder einen Periskopkegel und/oder ein Kamerasystem umfasst.

3. Biopsievorrichtung nach Anspruch 1, umfassend eine Vielzahl von Gewebehaltehohlräume zur Aufnahme einzelner Gewebeproben und wobei die Gewebeprobensammelvorrichtung und die Probenaufnahmevorrichtung bezüglich einander so beweglich sind, dass verschiedene, zu verschiedenen Zeiten gewonnene Gewebeproben in getrennten Hohlräumen der Vielzahl von Hohlräumen untergebracht werden können.

4. Biopsiesystem nach Anspruch 3, wobei die Probensammelvorrichtung eine drehbare Trommel umfasst, die die Vielzahl von Hohlräumen umfasst, die entlang ihres Umfangs angeordnet sind.

5. Biopsievorrichtung nach Anspruch 3, wobei das Inspektionssystem so angeordnet ist, dass es eine Inspektion einer Gewebeprobe nach Ausstoß der Gewebeprobe in einen der Hohlräume und nach Relativbewegung der Probenaufnahmevorrichtung und der Probensammelvorrichtung zum relativen Verschieben des einen Hohlraums, in dem die Gewebeprobe untergebracht ist, von der Position weg, in der die Gewebeprobe in den einen Hohlraum ausgestoßen worden ist, gestattet.

6. Biopsievorrichtung nach Anspruch 1, wobei die Probensammelvorrichtung in einer Einwegeinheit der Biopsievorrichtung umfasst ist.

7. Biopsievorrichtung nach Anspruch 4, wobei die Trommel um eine Rotationsachse drehbar ist, die im Wesentlichen parallel zu einer Längsachse der Hohlnadel verläuft.

8. Biopsievorrichtung nach einem der vorhergehenden Ansprüche, wobei die Gewebeprobensammelvorrichtung mindestens ein Schöpfelement (142) umfasst, das an ihrem äußeren Umfang angeordnet ist, wobei das Schöpfelement dazu angeordnet ist, Gewebe in der Probenaufnahmevorrichtung zu sammeln, wenn die Probenaufnahmevorrichtung in ihrer zweiten eingezogenen Position ist.

9. Biopsievorrichtung nach Anspruch 8, wobei die Probensammelvorrichtung zwischen einer ersten, angehobenen Position, in der das Schöpfelement einen Gewebeaufnahmebehälter der Probenaufnahmevorrichtung nicht in Eingriff nimmt, und einer zweiten, abgesenkten Position, in der das Schöpfelement den Gewebeaufnahmebehälter in Eingriff nimmt, beweglich ist.

10. Biopsievorrichtung nach Anspruch 8 oder 9, wobei das mindestens eine Schöpfelement mindestens eine Wand mindestens eine der Gewebehaltehohlräume bildet.

## Revendications

1. Dispositif de biopsie configuré de manière à collecter au moins un échantillon de tissu à partir du corps d'un être vivant, comprenant:
une aiguille creuse (110) présentant une partie d'extrémité distale apte à être introduite dans le corps;
un mécanisme de coupe pour découper ledit au moins un échantillon de tissu;
un dispositif de réception d'échantillon (108) pour recevoir ledit au moins un échantillon de tissu découpé, le dispositif de réception d'échantillon pouvant être reçu dans l'aiguille creuse et étant mobile dans celle-ci entre une première position étendue et une seconde position rétractée;
un dispositif de collecte d'échantillon de tissu (104) comportant une ou plusieurs cavité(s) de retenue de tissu;
un système d'éjecteur pour éjecter l'échantillon de tissu hors du dispositif de réception d'échantillon de tissu dans l'une desdites cavités de retenue de tissu, lorsque le dispositif de réception d'échantillon se trouve dans la seconde position rétractée;
un système d'inspection (178) permettant à un opérateur du dispositif de biopsie d'inspecter visuellement l'échantillon de tissu éjecté dans ladite cavité de retenue de tissu, pendant que ladite aiguille creuse reste dans le corps,
**caractérisé en ce que** le système d'éjecteur comprend une pluralité de broches mobiles (120) agencées avec des espacements mutuels le long d'un axe longitudinal de l'aiguille creuse, et dans lequel l'aiguille creuse comporte une pluralité de premières ouvertures (118) opposées auxdites broches, et dans lequel le dispositif de réception d'échantillon comporte une pluralité de deuxièmes ouvertures, qui sont alignées avec lesdites premières ouvertures, lorsque le dispositif de réception d'échantillon se trouve dans la seconde position rétractée, de telle sorte que lesdites broches puissent pénétrer dans lesdites premières et deuxièmes ouvertures pour éjecter l'échantillon de tissu.

2. Dispositif de biopsie selon la revendication 1, dans lequel le système d'inspection (178) comprend au moins un parmi un oculaire, un cône périscopique et un système de caméra.

3. Dispositif de biopsie selon la revendication 1, comprenant une pluralité de cavités de retenue de tissu pour recevoir des échantillons de tissu individuels, et dans lequel le dispositif de collecte d'échantillon de tissu et le dispositif de réception d'échantillon sont mobiles l'un par rapport à l'autre de telle sorte que des échantillons de tissu différents collectés à des moments différents puissent être disposés dans des cavités séparées de ladite pluralité de cavités.

4. Système de biopsie selon la revendication 3, dans lequel le dispositif de collecte d'échantillon comprend un tambour rotatif, qui comporte ladite pluralité de cavités agencées le long de sa circonférence.

5. Dispositif de biopsie selon la revendication 3, dans lequel le système d'inspection est agencé de manière à permettre l'inspection d'un échantillon de tissu après l'éjection de l'échantillon de tissu dans l'une desdites cavités et après un déplacement relatif du dispositif de réception d'échantillon et du dispositif de collecte d'échantillon afin de déplacer de façon relative ladite une première cavité contenant l'échantillon de tissu à distance de cette position dans laquelle l'échantillon de tissu a été éjecté dans ladite première cavité.

6. Dispositif de biopsie selon la revendication 1, dans lequel le dispositif de collecte d'échantillon est compris dans une unité jetable du dispositif de biopsie.

7. Dispositif de biopsie selon la revendication 4, dans lequel ledit tambour est rotatif autour d'un axe de rotation, qui est essentiellement parallèle à un axe longitudinal de l'aiguille creuse.

8. Dispositif de biopsie selon l'une quelconque des revendications précédentes, dans lequel ledit dispositif de collecte d'échantillon comprend au moins un élément de prélèvement (142) agencé sur sa circonférence extérieure, ledit élément de prélèvement étant agencé de manière à collecter un tissu dans le dispositif de réception d'échantillon, lorsque le dispositif de réception d'échantillon se trouve dans sa seconde position rétractée.

9. Dispositif de biopsie selon la revendication 8, lequel ledit dispositif de collecte d'échantillon de tissu est mobile entre une première position élevée, dans laquelle ledit élément de prélèvement n'engage pas un récipient de réception de tissu du dispositif de réception d'échantillon, et une seconde position abaissée, dans laquelle ledit élément de prélèvement engage ledit récipient de réception de tissu.

10. Dispositif de biopsie selon la revendication 8 ou 9, dans lequel ledit au moins un élément de prélèvement forme au moins une paroi d'au moins desdites cavités de retenue de tissu.
